(19) Europäisches Patentamt
European Patent Office
Office européen des brevets

(11) **EP 2 366 428 B1**

(12) **EUROPÄISCHE PATENTSCHRIFT**

(45) Veröffentlichungstag und Bekanntmachung des Hinweises auf die Patenterteilung:
**28.08.2013 Patentblatt 2013/35**

(51) Int Cl.:
***A61N 5/10*** *(2006.01)*

(21) Anmeldenummer: **10170091.2**

(22) Anmeldetag: **20.07.2010**

(54) **Verfahren zum Registrieren eines ersten Abbildungsdatensatzes zu einem zweiten Abbildungsdatensatz**

Method for registering an initial reproduction dataset to a second reproduction dataset

Procédé d'enregistrement d'un premier ensemble de données représentatives vers un second ensemble de données représentatives

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **07.09.2009 DE 102009040392**

(43) Veröffentlichungstag der Anmeldung:
**21.09.2011 Patentblatt 2011/38**

(73) Patentinhaber: **Siemens Aktiengesellschaft**
**80333 München (DE)**

(72) Erfinder: **Rietzel, Eike**
**64331 Weiterstadt (DE)**

(56) Entgegenhaltungen:
**EP-A1- 1 900 392        EP-A1- 1 911 493**
**WO-A1-2006/018761        US-A1- 2006 074 292**

- **MAINTZ A J B ET AL: "A survey of medical image registration", MEDICAL IMAGE ANALYSIS, OXFORD UNIVERSITY PRESS, OXOFRD, GB, Bd. 2, Nr. 1, 1. März 1998 (1998-03-01), Seiten 1-36, XP002604021, ISSN: 1361-8415**
- **JÄKEL O ET AL: "Relation between carbon ion ranges and x-ray CT numbers", MEDICAL PHYSICS, AIP, MELVILLE, NY, US, vol. 28, no. 4, 1 April 2001 (2001-04-01), pages 701-703, XP012011450, ISSN: 0094-2405, DOI: 10.1118/1.1357455**

EP 2 366 428 B1

**Beschreibung**

Verfahren zum Registrieren eines ersten Abbildungsdatensatzes zu einem zweiten Abbildungsdatensatz und Vorrichtung

Die Erfindung betrifft ein Verfahren zum Registrieren eines ersten Abbildungsdatensatzes zu einem zweiten Abbildungsdatensatz sowie eine Vorrichtung zur Durchführung eines derartigen Verfahrens. Registrierungsverfahren werden insbesondere in der medizinischen Bildgebung eingesetzt, um zwei Abbildungsdatensätze miteinander zu vergleichen. Insbesondere im Rahmen einer Strahlentherapie werden derartige Verfahren oftmals bei der Patientenpositionierung angewendet.

Die Strahlentherapie im Allgemeinen und die Partikeltherapie im Speziellen sind etablierte Verfahren zur Behandlung von Gewebe, insbesondere von Tumorerkrankungen. Bestrahlungsverfahren, wie sie in der Strahlentherapie eingesetzt werden, finden jedoch auch in nicht-therapeutischen Gebieten Anwendung. Hierzu gehören beispielsweise Forschungsarbeiten im Rahmen der Strahlentherapie, die an nicht-lebenden Phantomen oder Körpern durchgeführt werden, Bestrahlungen von Materialien, etc.

Bei der Strahlentherapie wird ein Strahl erzeugt, beispielsweise ein Röntgenstrahl, ein Elektronenstrahl oder ein Partikelstrahl aus geladenen Teilchen wie Protonen, Kohlenstoffionen oder andere geladene Teilchen, und auf das zu bestrahlende Objekt gerichtet. Um den Bestrahlungserfolg zu gewährleisten, ist es wichtig, dass das zu bestrahlende Objekt möglichst genau in Bezug auf den Strahl positioniert wird.

Im Rahmen der Strahlentherapie wird dies oftmals dadurch erreicht, dass die Therapieplanung an einem Abbildungsdatensatz vorgenommen wird und dass im Vorfeld einer Therapiesitzung ein Vergleichsdatensatz aufgenommen wird. Beide Datensätze werden zueinander registriert, und aus dieser Registrierung werden Positionsinformationen ermittelt, mit denen der zu bestrahlende Patient relativ zu dem Strahl positioniert werden kann. Dies dient dazu, dass eine nachfolgende Bestrahlung möglichst genau der geplanten Bestrahlung entspricht.

Ein Überblick über bekannte Registrierungsverfahren ist z.B. in der Schrift J. B. A. Maintz and M. A. Viergever, "A survey of medical image regis-tration", Medical Image Analysis 2(1), pp. 1-36, 1998, gegeben.

Ein Registrierungsverfahren zur Anpassung eines Bestrahlungsfeldes ist aus der Patentanmeldung EP 1900392 A1 bekannt.

Es ist die Aufgabe der Erfindung, ein Verfahren zur Registrierung zweier Abbildungsdatensätze bereitzustellen, welches eine besonders genaue Positionierung eines zu bestrahlenden Objekts in Bezug auf einen Strahl ermöglicht. Weiterhin ist es die Aufgabe der Erfindung, eine Verwendung eines derartigen Verfahrens zur Positionierung eines zu bestrahlenden Objekts in Bezug auf einen Strahl bereitzustellen. Weiterhin ist es die Aufgabe der Erfindung, eine Vorrichtung zur Durchführung eines derartigen Verfahrens bereitzustellen.

Die Aufgabe der Erfindung wird gelöst durch ein Verfahren gemäß Anspruch 1, durch eine Verwendung eines derartigen Verfahrens gemäß Anspruch 12 sowie durch eine Vorrichtung gemäß Anspruch 13. Vorteilhafte Weiterbildungen finden sich in den Merkmalen der abhängigen Ansprüche.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Bei dem erfindungsgemäßen Verfahren zum Registrieren eines ersten Abbildungsdatensatzes zu einem zweiten Abbildungsdatensatz eines Objekts wird eine Reichweiteninformation ermittelt, welche die Reichweite eines Strahls in dem Objekt beschreibt, und zumindest ein Transformationsparameter ermittelt, welcher eine Registrierung des ersten Abbildungsdatensatzes zu dem zweiten Abbildungsdatensatz beschreibt, wobei die Ermittlung des zumindest einen Transformationsparameters unter Verwendung der ermittelten Reichweiteninformation erfolgt.

Dem erfindungsgemäßen Verfahren liegt die Idee zu Grunde, dass bei bisher angewendeten Registrierungsverfahren die Registrierung stets auf den Bildinformationen beruht, die in den Abbildungsdatensätzen hinterlegt sind. In vielen Fällen kann dies bereits ausreichend sein, um beispielsweise einen Patienten in hinreichender Genauigkeit relativ zu dem Strahl zu positionieren. Es wurde aber erkannt, dass die Bildinformation alleine nicht notwendigerweise zu der bestmöglichen Positionierung führt. Die Bildinformation alleine berücksichtigt noch nicht das Verhalten des Strahls in dem zu bestrahlenden Objekt - zumindest nicht in dem Umfang, wie die Bildinformation bei herkömmlichen Registrierungsverfahren verwendet wird.

Wenn der Registrierung lediglich die Bildinformationen zu Grunde liegen, kann beispielsweise der Fall eintreten, dass die Registrierung das Ergebnis liefert, dass die Bilddatensätze zueinander rotiert sind. Wenn nun eine Patientenpositionierung aufgrund dieser Registrierung durchgeführt würde, würde auch der Patient entsprechend rotiert werden. Dies könnte aber bewirken, dass nun im Eintrittskanal eines Strahls dichtes Gewebe zu liegen kommt, welches die Reichweite des Strahls erheblich beeinflussen würde. In diesem Falle würde eine Registrierung, welche lediglich auf der in den Abbildungsdatensätzen enthaltenen Bildinformationen beruht, zu einem unzureichenden Ergebnis führen.

Es wurde aber erkannt, dass das Verhalten des Strahls in dem zu bestrahlenden Objekt und die sich ergebende Reichweite eine weitere relevante Information ist, welche die Genaugkeit einer Bestrahlung beeinflusst.

Mit dem erfindungsgemäßen Verfahren wird eine Reichweiteninformation ermittelt, die diesen Sachverhalt widerspiegelt. Die Reichweiteninformation gibt an, wie stark ein Strahl in das zu bestrahlende Objekt eindringt oder wie stark ein Strahl durch das zu bestrahlende Objekt geschwächt wird. Dies ist insbesondere bei Partikelstrahlen relevant, da hier der Strahl abhängig von seiner Energie in eine bestimmte Tiefe eindringt und dann in einem relativ eng umschriebenen Bereich, der durch den so genannten Bragg-Peak gekennzeichnet ist, den Hauptteil seiner Energie abgibt.

Bei dem erfindungsgemäßen Verfahren bei der Ermittlung des zumindest einen Transformationsparameters, also bei der Registrierung der beiden Abbildungsdatensätze zueinander, können nicht nur die Bildinformationen, die in den Abbildungsdatensätzen hinterlegt sind, verwendet werden, sondern zusätzlich auch die Reichweiteninformation. Hierdurch ergibt sich insgesamt eine Registrierung der beiden Abbildungsdatensätze zueinander, welche die Bedürfnisse, die einer Strahlentherapie, insbesondere einer Partikeltherapie, zu Grunde liegen, besser berücksichtigt.

Die Reichweiteninformation wird z.B. aus der Bildinformation ermittelt. Wenn als Abbildungsdatensatz z.B. ein Computertomographie-Datensatz verwendet wird, kann die Reichweiteninformation ermittelt werden, indem bezogen auf eine Einstrahlrichtung die HU-Werte (HU für "hounsfield units") der Voxel, die in Einstrahlrichtung hintereinander liegen, aufsummiert werden.

In einer einfachen Ausgestaltung kann eine Reichweiteninformation dazu verwendet werden, die Bilddaten der Abbildungsdatensätze zu gewichten und auf diese Weise zur Ermittlung der Transformationsparameter, welche die Registrierung der Abbildungsdatensätze zueinander beschreibt, beizutragen. Dies ist vor allem dann vorteilhaft, wenn ein nicht-linearer Zusammenhang zwischen den Werten, welche die Bildinformation beschreiben, und der Reichweite eines Strahls gegeben ist. Vorzugsweise wird zu jedem der beiden Datensätze jeweils eine Reichweiteninformation ermittelt. Bei der Registrierung der zwei Abbildungsdatensätze zueinander werden dann ebenfalls die beiden Reichweiteninformationen miteinander verglichen.

Diese Reichweiteninformationen können dann hinsichtlich vorliegender Unterschiede in analoger Weise miteinander verglichen werden wie die Bildinformationen bei herkömmlichen Registrierungsverfahren.

In einer Ausführungsform der Erfindung ist es vorgesehen, bei der Ermittlung des zumindest einen Transformationsparameters eine Funktion zu verwenden, welche einen Unterschied, z.B. eine Differenz, zwischen den beiden Reichweiteninformationen beschreibt.

Es ist jedoch nicht zwingend notwendig, zu jedem der beiden Datensätze eine Reichweiteninformation zu ermitteln. Beispielsweise ist es auch denkbar, lediglich zu einem Datensatz eine Reichweiteninformation zu ermitteln und dann diese Reichweiteninformation dazu zu verwenden, um Bildbereiche in den Abbildungsdatensätzen bei der Registrierung unterschiedlich stark zu gewichten. Auf diese Weise kann z.B. berücksichtigt werden, dass bestimmte Bildbereiche nicht bedeutend sind für die Registrierung, da ein Teilchenstrahl gar nicht oder nur in abgeschwächter Weise zu den entsprechenden Regionen im Objekt vordringen würde.

In einer vorteilhaften Ausführungsform werden die Reichweiteninformationen bei einem Abbildungsdatensatz nicht für den gesamten Abbildungsdatensatz ermittelt, sondern lediglich für einen Teilbereich des Abbildungsdatensatzes. Auf diese Weise kann beispielsweise berücksichtigt werden, dass ein Partikelstrahl ein zu bestrahlendes Objekt nicht notwendigerweise durchdringt sondern nur bis zu einer gewissen Tiefe vordringt. Daher reicht es in diesem Fall, dass die Reichweiteninformationen nicht für den gesamten Abbildungsdatensatz ermittelt werden, sondern lediglich für einen Teilbereich, der in diesem Fall durch die Eindringtiefe des Partikelstrahls gegeben ist. Auf diese Weise lässt sich die Berechnung schneller durchführen. Die Reichweiteninformationen kann beispielsweise lediglich bis zu einer distalen Kante ermittelt werden, wobei die distale Kante durch eine maximale Eindringtiefe des Strahls gegeben ist.

In einer Ausführungsvariante kann eine Registrierungsmaske bestimmt werden, welche diejenigen Bereiche des oder der Abbildungsdatensätze kennzeichnet, die bei der Ermittlung der Transformationsparameter berücksichtigt werden sollen. Diese Registrierungsmaske kann dabei auch unter Berücksichtigung der Reichweiteninformation ermittelt werden.

Dies berücksichtigt die Tatsache, dass es oftmals besser ist, nicht den gesamten Abbildungsdatensatz der Registrierung zu Grunde zu legen, sondern lediglich einen Teilbereich. Bei der Bestrahlung der Prostata z.B. kann es vorteilhaft sein, lediglich die Prostata und den angrenzenden Bereich der Blase bzw. des Rektums der Registrierung zu Grunde zu legen. Auf diese Weise wird verhindert, dass Bereiche in den Abbildungsdatensätzen in die Bestimmung der Transformationsparameter einfließen, die auf die Position und/oder Lage des zu bestrahlenden Gebiets nur einen geringen Einfluss haben. Würden diese Bereiche bei der Bestimmung der Transformationsparameter berücksichtigt, könnte dies dazu führen, dass eine Positionierung des Objekts, die auf den Transformationsparametern beruht, eher zu einer schlechteren Dosisapplikation führt als zu einer besseren.

In einer vorteilhaften Ausführungsform umfasst die Reichweiteninformation eine Mehrzahl von Reichweiten-Teilinformationen, die jeweils unterschiedlichen Regionen in den Abbildungsdatensätzen, insbesondere einzelnen Voxeln, zugeordnet sind. Auf diese Weise kann unterschiedlichen Regionen oder gar einzelnen Voxeln jeweils eine Reichweiten-Teilinformationen zugeordnet werden, die angibt, welchen Einfluss die jeweilige Region bzw. das jeweilige Voxel auf die Reichweite eines Strahls hat.

Die Reichweiteninformation kann beispielsweise als wasseräquivalente Tiefe angegeben werden. Die wasseräquivalente Tiefe ist dabei ein bekanntes Maß, das insbesondere im Rahmen der Partikeltherapie verwendet wird. Ein Voxel kann beispielsweise durch die wasseräquivalente Tiefe charakterisiert werden. In diesem Fall gibt die wasseräquivalente Tiefe des Voxels an, welche Tiefe bzw. Strecke ein Partikelstrahl in einem homogenen Körper aus Wasser durchdringen muss, um gleich stark abgeschwächt zu werden wie durch das Voxel selbst.

Insbesondere kann die Reichweiteninformation bezogen auf eine Einstrahlrichtung ermittelt werden. In diesem Fall kann beispielsweise als Reichweiteninformation eine integrale wasseräquivalente Tiefe - bezogen auf die Einstrahlrichtung - angegeben werden.

Wenn beispielsweise einem Voxel oder einer Region in dem zu bestrahlenden Objekt eine integrale wasseräquivalente Tiefe zugeordnet wird, gibt dieses Maß an, welche Tiefe/Strecke ein Strahl in einem homogenen Körper aus Wasser durchdringen müsste, um gleich stark abgeschwächt zu werden wie in dem Objekt vom Eintrittsort in das Objekt bis zu dem Voxel bzw. zu der Region hin, das Voxel bzw. die Region eingeschlossen.

In einer vorteilhaften Ausführungsform wird, falls die Reichweiteninformation auf eine Einstrahlrichtung bezogen ermittelt wird, bei der Ermittlung der Transformationsparameter die Reichweiteninformation von neuem ermittelt, wenn eine Einstrahlrichtung geändert wird. Auf diese Weise kann das Verfahren auch dann verwendet werden, wenn die Transformationsparameter eine Änderung der Einstrahlrichtung zu lassen. Dies ist beispielsweise dann der Fall, wenn bei der Registrierung eine Rotation als Freiheitsgrad zugelassen wird. Nachdem die Transformationsparameter mit dem erfindungsgemäßen Verfahren ermittelt worden sind, können die Transformationsparameter dazu verwendet werden, eine Positionierung des zu bestrahlenden Objekts in Bezug auf den Strahl durchzuführen.

Die erfindungsgemäße Vorrichtung zur Registrierung eines ersten Abbildungsdatensatzes zu einem zweiten Abbildungsdatensatz weist eine Rechnereinheit auf, welche zur Durchführung eines der erfindungsgemäßen Verfahren ausgebildet ist.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:

Fig. 1 einen schematischen Überblick über eine Partikeltherapieanlage,

Fig. 2 eine Darstellung eines schematisierten, zweidimensionalen ersten Abbildungsdatensatzes,

Fig. 3 eine Darstellung eines schematisierten, zweidimensionalen zweiten Abbildungsdatensatzes mit räumlich leicht veränderter Lage der abgebildeten Strukturen,

Fig. 4 und Fig. 5 eine Darstellung der Voxel-Werte, welche Fig. 2 bzw. Fig. 3 entsprechen,

Fig. 6 und Fig. 7 eine Darstellung von integralen Reichweiteninformationen, die Fig. 2 bzw. Fig. 3 entsprechen,

Fig. 8 eine Darstellung einer Registrierungsmaske,

Fig. 9 eine Darstellung von Reichweiteninformationen, welche lediglich bis zu einer distalen Kante berechnet worden sind, und

Fig. 10 eine schematische Übersicht über einzelne Verfahrensschritte.

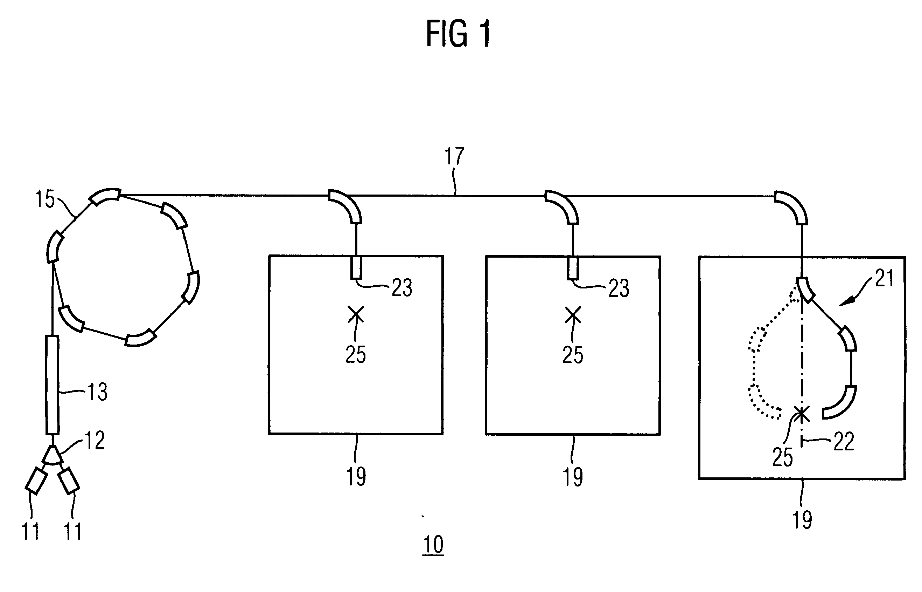

Figur 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Heliumionen, Kohlenstoffionen oder andere Teilchenarten wie Pionen eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Im Bestrahlungsraum 19 tritt der Partikelstrahl aus einem Strahlauslass 23 aus und trifft auf ein zu bestrahlendes Zielvolumen, das sich üblicherweise im Isozentrum 25 eines Bestrahlungsraums befindet.

Zur korrekten Positionierung eines Patienten im Bestrahlungsraum 19 ist es üblich, z.B. vor einer geplanten Bestrahlungssitzung die Anatomie des Patienten erneut abzubilden. Dieser Abbildungsdatensatz kann dann zu einem Planungs-CT registriert werden, um hieraus Steuerparameter zu ermitteln, mit denen eine Patientenpositioniervorrichtung den zu bestrahlenden Patienten passend zu einem Behandlungsstrahl ausrichtet, um eine möglichst genaue Dosisapplikation zu gewährleisten, falls anschließend tatsächlich die Bestrahlung stattfindet. Hierfür kann eine Vorrichtung in der Partikeltherapieanlage vorgesehen sein, die zur Durchführung eines Verfahrens gemäß der Erfindung ausgebildet ist.

Der anhand der Figur 1 dargestellte Grundaufbau einer Partikeltherapieanlage 10 ist beispielhaft für Partikeltherapieanlagen, kann aber auch hiervon abweichen.

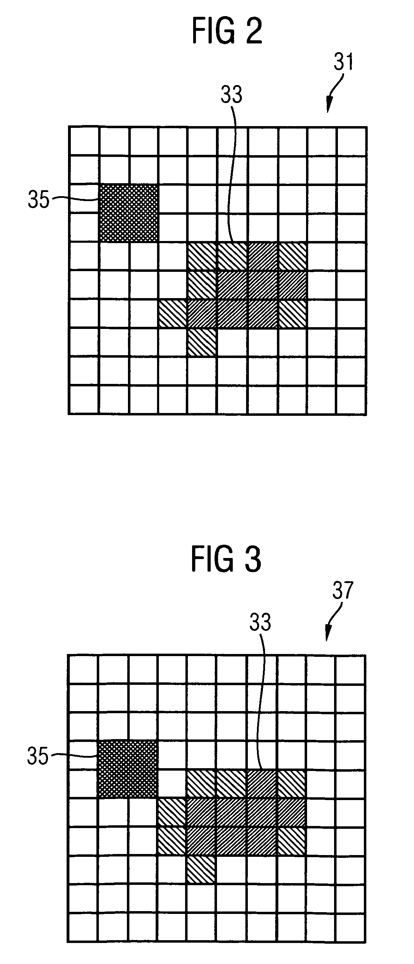

Anhand der nachfolgenden Figuren 2 bis 8 wird das der Erfindung zu Grunde liegende Konzept näher erläutert. Ausführungsformen der Erfindung können dabei in Zusammenhang mit einer Partikeltherapieanlage wie anhand von Fig. 1 dargestellt eingesetzt werden, aber auch mit anderen Bestrahlungsanlagen. Darüber hinaus sind Ausführungsformen der Erfindung nicht auf den Einsatz im Rahmen der Strahlentherapie beschränkt.

Fig. 2 ist dabei eine stark schematisierte Darstellung eines ersten Abbildungsdatensatzes 31. Zu sehen ist eine zweidimensionale Matrix aus Voxeln. In dieser Matrix ist ein zu bestrahlendes Zielvolumen 33 abgebildet und ein in der Nähe des Zielvolumens liegendes Gebiet 35, das Einfluss auf die Bestrahlung des Zielvolumens 33 haben kann. Fig. 3 zeigt die räumlichen Verhältnisse in einem zweiten Abbildungsdatensatz 37, der das Zielvolumen 33 und das in der Nähe des Zielvolumens liegende Gebiet 35 zu einem späteren Zeitpunkt abbildet. Zu sehen ist, dass sich die räumlichen Verhältnisse leicht geändert haben.

Der erste Abbildungsdatensatz 31 kann beispielsweise einer Bestrahlungsplanung zu Grunde liegen, der zweite Abbildungsdatensatz 37 kann beispielsweise für eine Positionierung des Patienten aufgenommen werden. Durch einen Vergleich des ersten Abbildungsdatensatzes 31 mit den zweiten Abbildungsdatensatz 37 können Parameter bestimmt werden, mit deren Hilfe sich das zu bestrahlende Objekt 33, 35 im Vorfeld eine Bestrahlung passend positioniert werden kann, so dass - falls anschließend tatsächlich eine Bestrahlung durchgeführt wird - die Bestrahlung möglichst konform zu der Bestrahlungsplanung durchgeführt werden kann.

Der Vergleich des ersten und des zweiten Abbildungsdatensatzes 31, 37 findet in Form eine Registrierung statt. Die Transformationsparameter, welche die Registrierung beschreiben, können dann zur Ermittlung der passenden Patientenpositionierung verwendet werden.

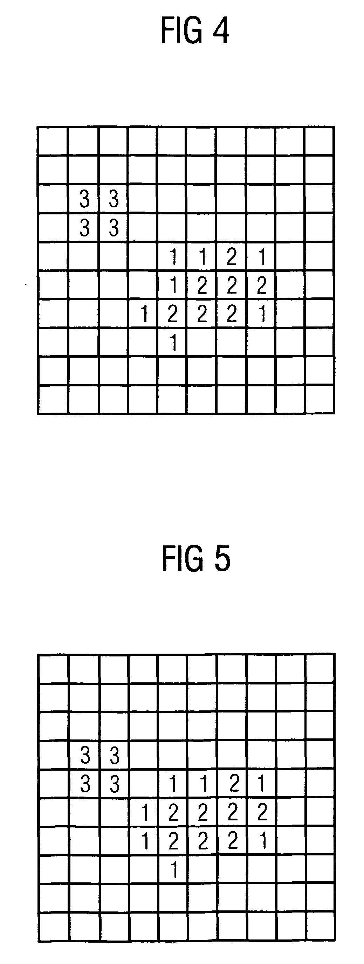

Fig. 4 und Fig. 5 ist eine Darstellung, in der den Voxeln Zahlenwerte zugeordnet sind. Die Zahlenwerte repräsentieren die Grauwerte der Voxel. Der Einfachheit und Übersichtlichkeit halber wurden hier die Zahlenwerte 1, 2, 3 gewählt, um die Voxel aus Fig. 2 bzw. Fig. 3 zu charakterisieren. Nicht explizit mit Zahlenwerten belegte Voxel entsprechen einem Zahlenwert von null. Wenn als Abbildungsdatensatz ein CT-Datensatz verwendet wird, können die Zahlenwerte z.B. die HU-Einheiten sein, welche die Dichte der Voxel des CT-Datensatzes charakterisieren.

Die Zahlenwerte aus Fig. 4 und Fig. 5 stellen letztlich Bildinformationen dar, welche in den Abbildungsdatensätzen 31, 37 hinterlegt sind. Zu erkennen ist, dass sich die Bildinformationen geringfügig zwischen den beiden Abbildungsdatensätzen 31, 37 unterscheiden. Die Unterschiede bestehen in diesem exemplarischen Beispiel darin, dass das Zielvolumen 33 eine geringfügige Vergrößerung aufweist, und dass weiterhin die Lage des Gebiets 35 in der Nähe des Zielvolumens um ein Voxel nach unten verschoben ist.

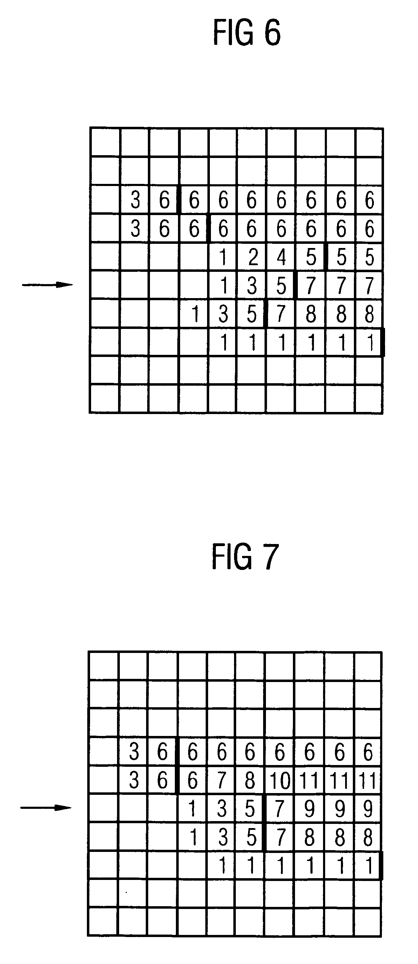

Aus der Bildinformation alleine geht noch nicht hervor, dass diese geringfügigen Änderungen einen vergleichsweise großen Effekt auf die Reichweite eines Strahls haben können. Dies wird anhand von Fig. 6 und Fig. 7 verdeutlicht.

In Fig. 6 und Fig. 7 sind Reichweiteninformationen dargestellt, die aus den in den Abbildungsdatensätzen hinterlegten Bildinformationen, vgl. Fig. 4 und Fig. 5, ermittelt worden sind.

Der Einfachheit halber wird hier angenommen, dass einem Voxel mit einem Zahlenwert von 1 in Fig. 4 oder Fig. 5 eine wasseräquivalente Tiefe von 1 entspricht, und dass ein linearer Zusammenhang zwischen den Zahlenwerten der Voxel aus Fig. 4 bzw. Fig. 5 und der wasseräquivalente Tiefe besteht. Dies bedeutet zum Beispiel, dass ein Voxel mit einem Zahlenwert von 3 eine wasseräquivalente Tiefe von 3 Einheiten aufweist.

In Fig. 6 und Fig. 7 sind als Vielzahl von Reichweiteninformationen jeweils die integrale wasseräquivalente Tiefe bezogen auf eine Einstrahlrichtung von links (angedeutet durch den Pfeil) pro Voxel angegeben. Voxel, die nicht mit Zahlen belegt sind, weisen eine wasseräquivalente Tiefe von 0 auf.

Es ist zu erkennen, dass sich die integrale Reichweiteninformation des ersten Abbildungsdatensatzes 31, abgebildet in Fig. 6, von der integralen Reichweiteninformation des zweiten Abbildungsdatensatzes 37, abgebildet in Fig. 7, deutlich stärker unterscheidet als die Bildinformationen allein.

Die integrale Reichweiteninformation hat jedoch für den Erfolg eine Bestrahlung einen großen Einfluss. So würde ein Partikelstrahl, wenn er die fünfte Zeile des ersten Abbildungsdatensatzes 31 von links bestrahlen würde, wesentlich weiter vordringen als bei einer Bestrahlung der fünften Zeile des zweiten Abbildungsdatensatzes 37. Vergleiche hierfür die Zahlenwerte in der fünften Zeile in Fig. 6 und Fig. 7. Wenn man in dieser Zeile lediglich die Bildinformationen betrachtet (Fig. 2 bis Fig. 5), fällt der Unterschied wesentlich geringer aus.

Wenn nun bei einer Registrierung der Abbildungsdatensätze 31, 37 zueinander, d.h. bei der Ermittlung der Transformationsparameter, die Reichweiteninformationen berücksichtigt werden, wirkt sich dies auf das Ergebnis aus. Falls beispielsweise die Registrierung dazu verwendet wird, eine Patientenpositionierung im Vorfeld einer Bestrahlungssitzung zu bestimmen, werden die Auswirkungen, die sich aus der Reichweite eines Partikelstrahls ergeben, wesentlich besser berücksichtigt.

Einige Voxel sind in Fig. 6 und Fig. 7 durch eine dicke Linie am rechten Rand des Voxels gekennzeichnet. Diese dicke Linie kennzeichnet beispielsweise eine distale Kante, welche die maximale Eindringtiefe eines Partikelstrahls angibt.

Wenn die maximale Energie des Partikelstrahls z.B. derart gewählt ist, dass der Partikelstrahl lediglich eine wasseräquivalente Tiefe von 5 Einheiten durchdringen kann, würde sich eine distale Kante wie in Figur 6 bzw. Fig. 7 dargestellt ergeben. In diesem Falle kann es angebracht sein, die Reichweiteninformationen lediglich vom Eintrittsort des Partikelstrahls bis zu der distalen Kante zu berechnen, da die Reichweiteninformationen von Voxeln, die in Strahlrichtung hinter der distalen Kante liegen, auf ein weiteres Eindringen des Partikelstrahls keinen Einfluss mehr haben.

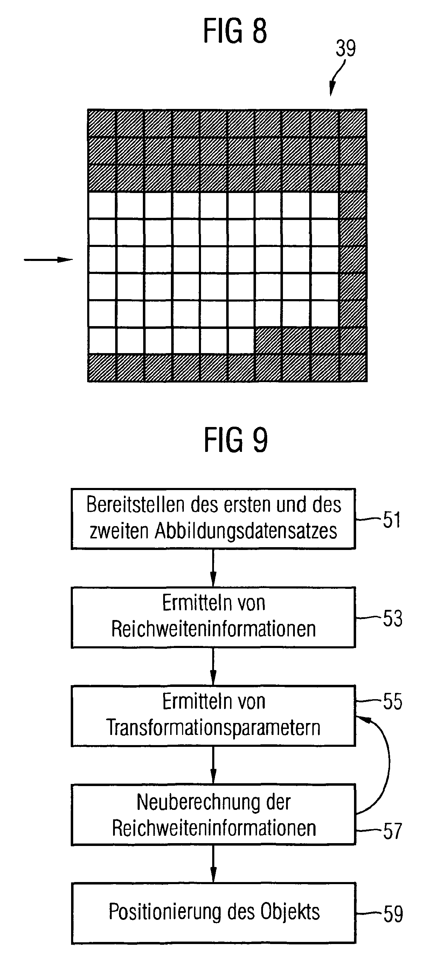

Fig. 8 zeigt eine Registrierungsmaske 39, die bei der Ermittlung der Transformationsparameter verwendet werden kann. Die Registrierungsmaske 39 gibt dabei die diejenigen Bildbereiche an, die in den Abbildungsdatensätzen 31, 37 zueinander registriert werden sollen. Mit der Registrierungsmaske kann beispielsweise gezielt das Zielvolumen bzw. das Zielvolumen umgebende Gebiet berücksichtigt werden. Zudem kann mit der Registrierungsmaske 39 der Einstrahlkanal berücksichtigt werden. Weiter entfernt vom Zielvolumen liegende Bereiche, die für die Lage des Zielvolumens unwesentlich sind und das Ergebnis der Registrierung eher verfälschen würden, können durch die Registrierungsmaske 39 ausgeschlossen werden.

Fig. 9 gibt einen schematischen Überblick über Verfahrensschritte, die bei der Durchführung einer Ausführungsform des Verfahrens durchgeführt werden.

Zunächst werden die Abbildungsdatensätze bereitgestellt, die zueinander registriert werden sollen (Schritt 51). In den Abbildungsdatensätzen kann beispielsweise das zu bestrahlende Objekt abgebildet sein. Anschließend wird zu jedem der beiden Abbildungsdatensätze Reichweiteninformationen ermittelt, die angeben, wie weit ein Strahl, z.B. ein Partikelstrahl in das Gewebe vordringt (Schritt 53). Anschließend erfolgt die Ermittlung der Transformationsparameter, welche die Registrierung der beiden Abbildungsdatensatz zueinander beschreibt, und zwar derart, dass zusätzlich zu den Bildinformationen die Reichweiteninformationen der beiden Abbildungsdatensätze berücksichtigt werden (Schritt 55). Falls bei der Ermittlung der Transformationsparameter eine Änderung der Einstrahlrichtung des Strahls vorgenommen wird, werden die Reichweiteninformationen von neuem ermittelt (Schritt 57). Die Transformationsparameter können mit der geänderten Einstrahlrichtung erneut berechnet werden. Nachdem die Transformationsparameter ermittelt worden sind, können z.B. hierauf basierend Steuerparameter ermittelt werden, welche zur Positionierung des zu bestrahlenden Objekts verwendet werden können (Schritt 59). Im Folgenden werden nähere Ausführungen dazu gemacht, wie Reichweiteninformationen, welche wie zuvor beschrieben ermittelt worden sind, bei der Ermittlung der Transformationsparameter einfließen können.

Wenn beispielsweise der erste Abbildungsdatensatz mit F bezeichnet wird und der zweite Abbildungsdatensatz mit G, geht es darum, eine geeignete Transformation T für den Abbildungsdatensatz F zu finden, sodass die Unterschiede zwischen dem transformierten Abbildungsdatensatz T(F) und G minimiert sind, wobei nun allerdings die Reichweiteninformation bei der Ermittlung berücksichtigt werden. Unter Minimierung wird hierbei nicht verstanden, dass zwangsläufigerweise das absolute Minimum gefunden werden muss. Der Unterschied kann auch beispielsweise iterativ verringert werden und das Iterationsverfahren abgebrochen werden, sobald eine bestimmte Bedingung erfüllt ist.

Die Reichweiteninformation, die einem der Bilddatensätze F und G zugeordnet sind, seien nachfolgend mit R(F) bzw. R(G) bezeichnet.

Das Auffinden der geeigneten Transformation kann dann z.B. als Optimierungsproblem gesehen werden, bei dem es darum geht

$$D(T(F), G) + a \cdot D(T(R(F)), R(G))$$

zu minimieren (falls D die Ungleichheit misst) bzw. zu maximieren (falls D die Gleichheit misst). Der erste Summand gibt dabei das herkömmliche Vorgehen bei bekannten Registrierungsverfahren an. Der zweite Summand führt nun neu die Reichweiteninformation ein. Mit dem Faktor a kann die Gewichtung festgelegt werden, mit der die Reichweiteninformationen berücksichtigt werden sollen.

Als Transformationsvorschriften T bzw. Funktion zum Messen des Unterschiedes D können bekannte bei der Bildregistrierung verwendete Funktionen T und D verwendet werden. Herkömmliche Registrierungsverfahren, welche in vorteilhafter Weise bei der Strahlentherapie eingesetzt werden, können nach oben angegebenem Muster modifiziert werden.

Ein Beispiel für ein bekanntes Registrierungsverfahren, das im Rahmen der Strahlentherapie zum Abgleich eines Portalbildes mit einem DRR (engl. für: "digitally reconstructed radiograph") vorgenommen wird, ist in der Schrift:

D.H. Hristov and B.G. Fallone, "A grey-level image alignment algorithm for registration of portal images and digitally reconstructed radio-graphs",

offenbart. Dieses Verfahren kann nach oben angegebenem Muster derart modifiziert werden, dass nun bei der Registrierung auch die Reichweiteninformation berücksichtigt wird. Da bei diesem Registrierungsverfahren auch Rotationen zugelassen werden, sind die integralen Reichweiteninformationen, die von der Einstrahlrichtung abhängen, immer dann neu zu berechnen, wenn bei der Optimierung ein anderer Einstrahlwinkel gewählt wird.

Weitere ähnliche Registrierungsverfahren zum Abgleich eines Portalbildes mit einem DRR sind in den Schriften:

Med. Phys. 23(7), Jan 1996 J. Moseley and P. Munro, "A semiautomatic method for registration of portal images", Med. Phys. 21(4), Apr 1994 und

L. Dong and A. L. Boyer, "A portal image alignment and patient setup verification procedure using moments and correlation techniques", Phys. Med. Biol. 41, 1996, pp 687-723 offenbart.

Auch wenn die oben angegebenen Registrierungsverfahren sich auf einen Abgleich von einem Portalbild mit einem DRR beziehen, kann ein derartiges Verfahren auch auf andere Abbildungsdatensätze angewendet werden. Die Verfahren können auf dreidimensionale oder vierdimensionale Abbildungsdatensätze verallgemeinert werden, es muss nicht notwendigerweise ein Portalbild mit einem DRR verglichen werden. Beispielsweise kann auch ein Cone-Beam-CT-Datensatz, der bei der Patientenpositionierung aufgezeichnet worden ist, mit einem Planungs-CT verglichen werden.

Einen Überblick über verschiedene Registrierungsverfahren gibt z.B. die Schrift:

J. B. A. Maintz and M. A. Viergever, "A survey of medical image regis-tration", Medical Image Analysis 2(1), pp. 1-36, 1998.

Ein Test, ob die Modifikation eines bekannten Registrierungsverfahrens auch z.B. für die Patientenpositionierung geeignet (ob z.B. der Wichtungsfaktor a passend gewählt worden ist), kann auf einfache Weise durchgeführt werden, indem das Verfahren an einer oder mehreren virtuellen Situationen (Planungs-CT bzw. Bilddatensatz zur Positionierung eines virtuellen Patienten) getestet wird, der virtuelle Patient anschließend gemäß der ermittelten Transformationsvorschrift "virtuell" positioniert wird und die Bestrahlung basierend auf der virtuellen Positionierung simuliert wird. Die "virtuell" applizierte Bestrahlung lässt sich dann mit einer vorgegebenen Zieldosis vergleichen. Auf diese Weise kann festgestellt werden, ob sich die Modifikation eines bekannten Registrierungsverfahrens dahingehend, dass nun auch die Reichweiteninformation berücksichtigt wird, auch tatsächlich zur Verbesserung einer Dosisdeposition führen würde.

Bezugzeichenliste

10   Partikeltherapieanlage

11   Ionenquelle

12   Schaltmagnet

13   Vorbeschleuniger

15   Beschleuniger

17   Hochenergiestrahl-Transportsystem

19   Bestrahlungsraum

21   Gantry

22   Achse

23   Strahlauslass

25   Isozentrum

31   erster Abbildungsdatensatz

33   Zielvolumen

35   Gebiet

37   zweiter Abbildungsdatensatz

39   Registrierungsmaske

51   Schritt 51

53   Schritt 53

55   Schritt 55

57   Schritt 57

59   Schritt 59

**Patentansprüche**

1.  Verfahren zum Registrieren eines ersten Abbildungsdatensatzes (31) zu einem zweiten Abbildungsdatensatz (37) eines Objekts (33, 35),
    bei dem eine Reichweiteninformation ermittelt wird, welche die Reichweite eines Strahls in dem Objekt (33, 35) beschreibt, und
    bei dem zumindest ein Transformationsparameter ermittelt wird, welcher eine Registrierung des ersten Abbildungsdatensatzes (31) zu dem zweiten Abbildungsdatensatz (37) beschreibt, wobei die Ermittlung des zumindest einen Transformationsparameters unter Verwendung der ermittelten Reichweiteninformation erfolgt,
    **dadurch gekennzeichnet,**
    **dass** die Reichweiteninformation bezogen auf eine Einstrahlrichtung ermittelt wird, wobei als Reichweiteninformation eine integrale wasseräquivalente Tiefe verwendet wird.

2.  Verfahren nach Anspruch 1, wobei
    bei dem zu jedem der beiden Abbildungsdatensätze (33, 35) jeweils eine Reichweiteninformation ermittelt wird.

3.  Verfahren nach Anspruch 2, wobei
    die Ermittlung des zumindest einen Transformationsparameters unter Verwendung einer Funktion erfolgt, welche einen Unterschied zwischen den beiden Reichweiteninformationen beschreibt.

4.  Verfahren nach einem der Ansprüche 1 bis 3,
    wobei die Reichweiteninformation lediglich für einen Teilbereich der Abbildungsdatensätze (31, 37) ermittelt wird.

5.  Verfahren nach eine der Ansprüche 1 bis 4,
    wobei die Reichweiteninformation lediglich bis zu einer distalen Kante, welche durch eine maximale Eindringtiefe des Strahls gegeben ist, ermittelt wird.

6. Verfahren nach einem der Ansprüche 1 bis 5,
   wobei eine Registrierungsmaske (39) bestimmt wird, welche die Bereiche kennzeichnet, die bei der Ermittlung der Transformationsparameter berücksichtigt werden.

7. Verfahren nach einem der Ansprüche 1 bis 6,
   wobei die Reichweiteninformation eine Mehrzahl von Reichweiten-Teilinformationen umfasst, die jeweils unterschiedlichen Regionen in einem oder beiden Abbildungsdatensätzen (31, 37), insbesondere einzelnen Voxeln, zugeordnet sind.

8. Verfahren nach einem der Ansprüche 1 bis 7,
   wobei bei der Ermittelung der Transformationsparameter eine Neuermittlung der Reichenweiteninformation erfolgt, wenn eine Einstrahlrichtung geändert wird.

9. Verwendung eines Verfahrens nach einem der Ansprüche 1 bis 8 zur Positionierung eines zu bestrahlenden Objekts (33, 35) relativ zu dem Strahl.

10. Vorrichtung zur Registrierung eines ersten Abbildungsdatensatzes zu einem zweiten Abbildungsdatensatz, umfassend eine Rechnereinheit, die zur Durchführung eines Verfahrens nach einem der Ansprüche 1 bis 8 ausgebildet ist.


**Claims**

1. Method for registering a first imaging data set (31) with a second imaging data set (37) of an object (33, 35),
   wherein range information is determined which describes the range of a beam in the object (33, 35), and
   wherein at least one transformation parameter is determined which describes a registration of the first imaging data set (31) with the second imaging data set (37), wherein the determination of the at least one transformation parameter is carried out using the determined range information,
   **characterised in that**
   the range information is determined with respect to a beam direction, wherein an integral water equivalent depth is used as range information.

2. Method according to claim 1, wherein
   range information is determined for each of the two imaging data sets (33, 35) respectively.

3. Method according to claim 2, wherein
   the determination of the at least one transformation parameter is carried out using a function which describes a difference between the two sets of range information.

4. Method according to one of claims 1 to 3,
   wherein the range information is determined only for a partial area of the imaging data sets (31, 37).

5. Method according to one of claims 1 to 4,
   wherein the range information is determined only up to a distal edge which is given by a maximum depth of penetration of the beam.

6. Method according to one of claims 1 to 5,
   wherein a registration mask (39) is defined which identifies the areas that are taken into consideration during the determination of the transformation parameters.

7. Method according to one of claims 1 to 6,
   wherein the range information comprises a plurality of range information subunits which are each associated with different regions in one or both imaging data sets (31, 37), in particular in individual voxels.

8. Method according to one of claims 1 to 7,
   wherein the range information is determined anew during the determination of the transformation parameters if a beam direction is changed.

9. Use of a method according to one of claims 1 to 8 for positioning an object to be irradiated (33, 35) relative to the beam.

10. Device for registering a first imaging data set with a second imaging data set, comprising a processing unit which is designed in order to execute a method according to one of claims 1 to 8.

## Revendications

1. Procédé de recalage d'un premier ensemble de données d'image (31) par rapport à un second ensemble de données d'image (37) d'un objet (33, 35), selon lequel une information de portée est déterminée, laquelle décrit la portée d'un rayon dans l'objet (33, 35) et
au moins un paramètre de transformation est déterminé, lequel décrit un recalage du premier ensemble de données d'image (31) par rapport au second ensemble de données d'image (37), la détermination de l'au moins un paramètre de transformation s'effectuant en utilisant l'information de portée déterminée,
**caractérisé en ce que** l'information de portée est déterminée en référence à une direction de rayonnement, une profondeur intégrale équivalente eau étant utilisée en tant qu'information de portée.

2. Procédé selon la revendication 1, selon lequel une information de portée est déterminée respectivement pour chacun des deux ensembles de données d'image (33, 35).

3. Procédé selon la revendication 2, la détermination de l'au moins un paramètre de transformation s'effectuant en utilisant une fonction qui décrit une différence entre les deux informations de portée.

4. Procédé selon l'une des revendications 1 à 3, l'information de portée étant déterminée uniquement pour une zone partielle des ensembles de données d'image (31, 37).

5. Procédé selon l'une des revendications 1 à 4, l'information de portée étant déterminée uniquement jusqu'à un bord distal qui est donné par une profondeur de pénétration maximale du rayon.

6. Procédé selon l'une des revendications 1 à 5, un masque de recalage (39) étant déterminé, lequel marque les zones qui sont prises en compte pour la détermination des paramètres de transformation.

7. Procédé selon l'une des revendications 1 à 6, l'information de portée comprenant une pluralité d'informations partielles de portée qui sont associées respectivement à différentes régions dans un ou dans les deux ensembles de données d'image (31, 37), et plus particulièrement à des voxels individuels.

8. Procédé selon l'une des revendications 1 à 7, une nouvelle détermination de l'information de portée ayant lieu, lors de la détermination des paramètres de transformation, lorsqu'une direction de rayonnement est modifiée.

9. Utilisation d'un procédé selon l'une des revendications 1 à 8 pour positionner un objet à irradier (33, 35) relativement au rayon.

10. Dispositif de recalage d'un premier ensemble de données d'image par rapport à un second ensemble de données d'image, comprenant une unité de calcul qui est réalisée pour exécuter un procédé selon l'une des revendications 1 à 8.

# FIG 1

...

# FIG 2

# FIG 3

# FIG 4

# FIG 5

EP 2 366 428 B1

# FIG 6

| | | | | | | | | | |
|---|---|---|---|---|---|---|---|---|---|
| | 3 | 6 | 6 | 6 | 6 | 6 | 6 | 6 | 6 |
| | 3 | 6 | 6 | 6 | 6 | 6 | 6 | 6 | 6 |
| | | | | 1 | 2 | 4 | 5 | 5 | 5 |
| → | | | | 1 | 3 | 5 | 7 | 7 | 7 |
| | | | 1 | 3 | 5 | 7 | 8 | 8 | 8 |
| | | | | 1 | 1 | 1 | 1 | 1 | 1 |

# FIG 7

| | | | | | | | | | |
|---|---|---|---|---|---|---|---|---|---|
| | 3 | 6 | 6 | 6 | 6 | 6 | 6 | 6 | 6 |
| | 3 | 6 | 6 | 7 | 8 | 10 | 11 | 11 | 11 |
| → | | | 1 | 3 | 5 | 7 | 9 | 9 | 9 |
| | | | 1 | 3 | 5 | 7 | 8 | 8 | 8 |
| | | | | 1 | 1 | 1 | 1 | 1 | 1 |

14

# FIG 8

39

# FIG 9

| | |
|---|---|
| Bereitstellen des ersten und des zweiten Abbildungsdatensatzes | 51 |
| Ermitteln von Reichweiteninformationen | 53 |
| Ermitteln von Transformationsparametern | 55 |
| Neuberechnung der Reichweiteninformationen | 57 |
| Positionierung des Objekts | 59 |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1900392 A1

**In der Beschreibung aufgeführte Nicht-Patentliteratur**

- **SCHRIFT J. B. A. MAINTZ ; M. A. VIERGEVER.** A survey of medical image regis-tration. *Medical Image Analysis,* 1998, vol. 2 (1), 1-36
- **D.H. HRISTOV ; B.G. FALLONE.** *A grey-level image alignment algorithm for registration of portal images and digitally reconstructed radio-graphs*
- *Med. Phys.,* Januar 1996, vol. 23 (7
- **J. MOSELEY ; P. MUNRO.** A semiautomatic method for registration of portal images. *Med. Phys.,* April 1994, vol. 21 (4
- **L. DONG ; A. L. BOYER.** A portal image alignment and patient setup verification procedure using moments and correlation techniques. *Phys. Med. Biol.,* 1996, vol. 41, 687-723
- **J. B. A. MAINTZ ; M. A. VIERGEVER.** A survey of medical image regis-tration. *Medical Image Analysis,* 1998, vol. 2 (1), 1-36